# EUROPÄISCHE PATENTSCHRIFT

(11) **EP 0 818 296 B1**
(45) Veröffentlichungstag und Bekanntmachung des Hinweises auf die Patenterteilung: **25.09.2002**
(21) Anmeldenummer: 97111271.9
(22) Anmeldetag: 04.07.1997
(51) Int. Cl.: B29C 45/32, A61M 5/34

(54) **Spritzgussteil und Vorrichtung zu dessen Herstellung**
Injection moulded object and apparatus for manufacturing the same
Objet moulé par injection et dispositif pour sa fabrication

(30) Priorität: 11.07.1996 DE 19627948
(43) Veröffentlichungstag der Anmeldung: 14.01.1998
(73) Patentinhaber: Sarstedt AG & Co., 51588 Nümbrecht (DE)
(72) Erfinder: Sarstedt, Walter, 51588 Nümbrecht (DE)
(74) Vertreter: Valentin, Ekkehard, Dipl.-Ing.

(56) Entgegenhaltungen:
- EP-A- 0 598 169
- DE-U- 8 902 045
- FR-A- 2 294 041
- GB-A- 2 277 685

## Beschreibung

Die Erfindung betrifft eine Vorrichtung zum Herstellen von Spritzgußteilen, die mit mindestens zwei seitlich abstehenden Vorsprüngen versehen sind, umfassend mindestens ein Werkzeug, das in zwei zwischen jeweils einer feststehenden und einer beweglichen Formbacke nebeneinanderliegenden Reihen angeordnete Formnester aufweist, sowie ein mit dieser Vorrichtung erhältliches Spritzgußteil.

Beim Herstellen von Formteilen durch Spritzgießen bereitet in der Regel die Entformung des fertiggespritzten Teiles bzw. Produktes nicht unerhebliche Probleme. Dies vor allem dann, wenn das Spritzgußteil mehrere Vorsprünge aufweist, von denen mindestens zwei diametral zueinander angeordnet sind, bzw. dessen Herstellung ein Formnest bzw. einen -hohlraum in mehr als einer Formbacke erfordert. Das ist z.B. dann der Fall, wenn bei einer aus der DE 30 49 503 C bekannten Blutentnahmevorrichtung eine stabilere Verbindung zwischen einer auf einen Ansatz bzw. Dom einer ein Blutentnahmeröhrchen verschließenden Kappe und einem eine Kanüle tragenden Halter erreicht werden soll. Eine solche stabile Verbindung wird beispielsweise weiterhin beim Anbringen eines Staubsaugerschlauches an das Gerätegehäuse gewünscht. So ist es bei Industriesaugern bekannt, am Außenmantel eines verdrehbar auf dem Schlauch befestigten Klemmstückes eine oder mehrere Nocken vorzusehen, die in korrespondierende Ausnehmungen des Gerätegehäuses eingreifen und sich dort verrasten lassen.

Die bei der bekannten Blutentnehmevorrichtung das Entnahmeröhrchen an seinem vorderen Ende abschließende Kappe besitzt einen zylindrischen, in axialer Richtung vorstehenden Dom. Der Dom besitzt an seinem vorderen Ende einen durchstechbaren Verschlußstopfen, der auf einer mit einer Mittelbohrung versehenen Stirnplatte des Doms aufliegt und von einem umgebördelten Kragen gehalten wird. Die rohrförmige Führungshülse, die hier an ihrem vorderen Ende in einem Halter eine doppelendige, beiderseits mit einer Schneidkante versehene Kanüle trägt, deren aus der Führungshülse vorstehendes Ende zur Einführung in eine Vene dient, während ihr hinteres Ende so weit in die Führungshülse hineinragt, daß es beim Ansetzen der Führungshülse an das Entnahmeröhrchen den Verschlußstopfen durchsticht, ist axial verschiebbar und drehbar auf dem Dom angeordnet. Das hintere, in die Führungshülse hineinragende Kanülenende wird von einem sackartigen Schlauch (Ventilgummi) solcher Länge eingehüllt, daß die Schneidkante des hinteren Kanülenendes bei gestrecktem Schlauch noch nicht dessen Boden berührt.

Zur Verbindung des die Doppelkanüle aufweisenden Halters mit dem Dom ist letzterer mit einem seitlich vorstehenden Haltenocken ausgebildet, dem Axialschlitze in der Führungshülse des Halters zugeordnet sind. Mittels des in einen der über den Umfang verteilten Axialschlitze eingeführten Haltenockens läßt sich eine bajonettverschlußartige Drehverriegelung der dann in lockerer Fassung auf dem Dom sitzenden Führungshülse erreichen. Eine derartige Drehverriegelung gewährleistet einen zwar sicheren, allerdings labilen Zusammenhalt der zusammengefügten Teile der Blutentnahmevorrichtung. Es hat sich gezeigt, daß bei z.B. drei von dem Dom seitlich abstehenden Vorsprüngen bzw. Haltenocken eine stabile Verbindung zwischen der Führungshülse und dem Ansatz bzw. Dom vorliegt.

Eine solche Mehrfach-Nockenanordnung am Dom der Kappe eines Blutentnahmeröhrchens bereitet im Gegensatz zur Herstellung einer Blutentnahmeröhrchen-Kappe mit lediglich einem abstehenden Haltenocken nicht unerhebliche Probleme. Der seitliche Nocken am Dom der bekannten Kappe wird durch eine Nebenkavität des eigentlichen Formhohlraumes bzw. -nestes in einer seitlich verschiebbaren Backe eines Backenwerzeuges vorgegeben. Um die durch Spritzgießen hergestellte, den abstehenden Nocken aufweisende Kappe entformen zu können, wird das Formnest in zwei komplementären Hälften von korrespondierenden Formbacken eines zweiteiligen Backenwerkzeuges vorgesehen. Die Trennebene verläuft bei diesen symmetrischen Teilen vorzugsweise genau in der Mitte. Während die eine Formbacke fest in dem Werkzeugaufbau angeordnet ist, läßt sich die sowohl die Kavität für den Nocken als auch den komplementären, hälftigen Teil-Formhohlraum aufweisende zweite Formbacke seitlich wegbewegen, so daß das Spritzgußteil ungehindert entformt werden kann.

Damit sich in einem Arbeitsgang mehrere der einen seitlichen Nocken aufweisenden Kappen gleichzeitig spritzgießen lassen, sind Werkzeuge bekannt, die in einem Werkzeug mehrere Formnester in zwei Reihen aufweisen. Ein hierzu eingesetztes Standard-Backenwerkzeug besitzt ein zwischen zwei beweglichen, äußeren Formbacken eingeschlossenes Werkzeugteil. Die eine Hälfte des Formnestes für die Kappe befindet sich jeweils in dem stationären Werkzeugteil und die andere, komplementäre Hälfte in der beweglichen Formbacke, in der auch die Kavität für den Nocken ausgebildet ist. Die Formnester für die Halter lassen sich in diesem Fall in zwei parallelen Reihen im Abstand nebeneinanderliegend vorsehen, wobei die Trennebene zwischen jeweils den beweglichen Formbacken und dem komplementären, stationären mittleren Werkzeugteil liegt. Durch die Anordnung der Kavität für die Haltenocken in den beweglichen Formbacken werden die Haltenocken beim Auffahren des Werkzeuges entformt, so daß sich anschließend das komplette Spritzgußteil auswerfen läßt.

Ein solcher dreiteiliger, das Spritzgießen von Fertigprodukten in zwei nebeneinanderliegenden Reihen ermöglichender bekannter Werkzeugaufbau versagt allerdings dann, wenn Spritzgußteile - wie die beschriebenen Halter - mit mehreren um 90^{o} versetzten Haltenocken hergestellt werden, da die in dem stationären, feststehenden Werkzeugteil vorgesehenen Haltenocken beim Auswerfen abgerissen bzw. -geschert würden. Kappen von Blutentnahmevorrichtungen mit z.B. drei seitlichen Haltenocken wurden deshalb in einem lediglich aus zwei Formbacken bzw. -hälften bestehenden Werkzeug spritzgegossen, die voneinander weg verfahrbar ausgebildet sind. Auf diese Weise ist zwar eine Entformung der Spritzgußteile möglich, jedoch lassen sich die Formnester für die Spritzgußteile nur noch in einer Reihe vorsehen, wobei die komplementären Hälften der Formnester zu beiden Seiten der Trennebene zwischen den beiden Werkzeugen liegen; gegenüber dem bekannten dreiteiligen Werkzeugaufbau wird durch diese Maßnahme die Kapazität, d.h. der Ausstoß von Spritzgußteilen pro Arbeitsgang halbiert.

Der Erfindung liegt die Aufgabe zugrunde, eine Vorrichtung der eingangs genannten Art zu schaffen, mit der sich mindestens zwei seitlich abstehende Vorsprünge aufweisenden Spritzgußteile in zwei nebeneinanderliegenden Reihen herstellen lassen, und ein mit dieser Vorrichtung insbesondere als Verschlußkappe für ein Entnahmeröhrchen einer Blutentnahmevorrichtung erhältliches Spritzgußteil vorzuschlagen.

Die erste Teilaufgabe wird mit einer Vorrichtung erfindungsgemäß dadurch gelöst, daß den Vorsprüngen Kavitäten ausschließlich in den beweglichen Formbacken zugeordnet sind, wobei die Kavität wenigstens des einen der Vorsprünge einer in den Trennebenen zwischen den Formbacken von jeweils der feststehenden Formbacke begrenzten Teilkontur einer üblichen vollständigen Kavität entspricht.

Der Erfindung liegt hierbei die Überlegung zugrunde, entweder alle oder zumindest einen bzw. zwei der Vorsprünge nicht mehr vollständig, sondern nur zur Hälfte auszubilden und damit beispielsweise zwei zueinander im wesentlichen diametral angeordnete Teilkavitäten zu ermöglichen, die im Vergleich zu einer vollständigen Kavität jeweils nur zur Hälfte ausgebildet sind und sich ausschließlich in den beweglichen Formbacken vorsehen lassen. Die somit zu erzielenden Spritzgußteile können daher - vorgegeben durch die Kavitäten - zwei in entgegengesetzter Richtung abstehende Halbform-Vorsprünge besitzen, die entlang ihrer axialen Mittelebene aufgrund der durch die feststehende Formbacke begrenzten Kavitäten eine ebene Abschlußfläche aufweisen, während ihre übrige Kontur sphärisch sein kann, so daß z.B. zwei sich zu Verriegelungszwecken einander ergänzende Halbzylinder als Vorsprünge bzw. Haltenocken vorliegen. Bei anderen möglichen Spritzgußteilen kann zwischen den beiden zueinander diametralen, halb- bzw. teilsymmetrischen Vorsprüngen ein mittiger, d.h. zu den beiden halbsymmetrischen Vorsprüngen um 90° versetzter, voll ausgeformter bzw. vollsymmetrischer Vorsprung vorgesehen sein, oder ein halbsymmetrischer Vorsprung läßt sich mit einem dazu rechtwinklig angeordneten vollsymmetrischen Vorsprung kombinieren; weiterhin ist es natürlich bei einer Anordnung mit drei Nocken auch möglich, alle Nocken ausschließlich als Halb- bzw. Teilnocken auszubilden. Bestimmend für alle Varianten ist stets die freie Entformbarkeit. Es weist somit stets wenigstens eine der die Form der Vorsprünge bestimmenden Kavitäten eine vorzugsweise einer Symmetriehälfte der Kavität eines üblicherweise vollständig ausgebildeten Vorsprungs entsprechende Kontur auf.

Es lassen sich daher Spritzgußteile mit mindestens zwei - vorzugsweise drei um jeweils 90° versetzten - Vorsprünge in zwei Reihen auf einem dreiteiligen Bakkenwerkzeug herstellen, ohne daß das Entformen der Spritzgußteile beeinträchtigt wird, denn nach dem Wegfahren der beiden beweglichen Formbacken werden sämtliche Vorsprünge freigegeben, so daß sich die kompletten Spritzgußteile entformen, d.h. ungehindert auswerfen lassen. Bei paralleler Anordnung von mehreren der vorbeschriebenen Werkzeuge läßt sich die Fertigungskapazität entsprechend erhöhen. Hierbei ist lediglich darauf zu achten, daß jeweils zwischen zwei benachbarten Werkzeugen ein ausreichender Freiraum für die beweglichen Formbacken eingehalten wird.

Wenn es sich bei den Vorsprüngen um zylindrische Ansätze handelt, besitzt z.B. nur der eine Ansatz eine volle Kreisform, während der bzw. die beiden zusätzlichen anderen Ansätze bzw. Vorsprünge nunmehr die Form eines Halbkreiszylinders besitzen. Die äußere Kontur der Vorsprünge ist im übrigen beliebig wählbar, d.h. sie kann von der vorbeschriebenen Kreisform abweichen und beispielsweise oval oder vieleckig sein.

Zur Lösung der zweiten Teilaufgabe wird für eine Verschlußkappe eines Blutentnahmeröhrchens einer Blutentnahmevorrichtung vorgeschlagen, daß eine das Vorderende des Entnahmeröhrchens der Blutentnahmevorrichtung temporär verschließende Kappe zur Festlegung eines aufsteckbaren, eine Kanüle tragenden Halters zumindest zwei diametral zueinander angeordnete, als halbe Vorsprünge ausgebildete Haltenocken aufweist.

Die Vorsprünge können um 90° versetzt auf einer Mantelhälfte der Kappe angeordnet sein, von denen der erste Vorsprung einen Vollnocken darstellt und die beiden anderen, zueinander diametralen zweiten und dritten Vorsprünge als Halbnocken ausgebildet sind, die der axial in der Mittenebene durchtrennten Symmetriehälfte des Vollnockens entsprechen. Wie sich anhand von Versuchen überraschend bestätigt hat, läßt sich ein sicherer Sitz und eine stabile Verbindung eines eine Kanüle tragenden Halters auf dem Dom einer Verschlußkappe eines Blutentnahmeröhrchens erreichen, wenn der Halter mit entsprechenden Schlitzen bajonettartig mit der mittleren Vollnocke und den beiden äußeren als Halbnocken ausgebildeten Vorsprünge verriegelt wird; die stabile Verbindung wird durch die lediglich halbe Ausbildung der beiden äußeren Vorsprünge nicht beeinträchtigt. Das gilt gleichermaßen für eine Kappe, bei der zwei diametral zueinander angeordnete Vorsprünge als Halbnocken ausgebildet sind; d.h. ein Vollnocken ist hier nicht vorhanden.

Weitere Merkmale und Vorteile der Erfindung ergeben sich aus den Patentansprüchen und der nachfolgenden Beschreibung, in der in den Zeichnungen schematisch dargestellte Ausführungsbeispiele des Gegenstandes der Erfindung näher erläutert sind. Es zeigen:
- Fig.1a, 1b: eine bekannte Verschlußkappe, in der Gesamtansicht (Fig. 1a) und in der Draufsicht (Fig. 1b) gezeigt;
- Fig. 2a, 2b: eine gemäß einem bevorzugten Ausführungsbeispiel mit drei um 90° versetzt zueinander angeordneten Haltenocken ausgebildete Verschlußkappe für ein Blutentnahmeröhrchen, in der Gesamtansicht (Fig. 2a) und in der Draufsicht (Fig. 2b) gezeigt; und
- Fig. 3: einen Werkzeugaufbau mit zwei parallel nebeneinander angeordneten Werkzeugen zum Herstellen von in den Fig. 2a, 2b gezeigten Verschlußkappen, schematisch dargestellt.

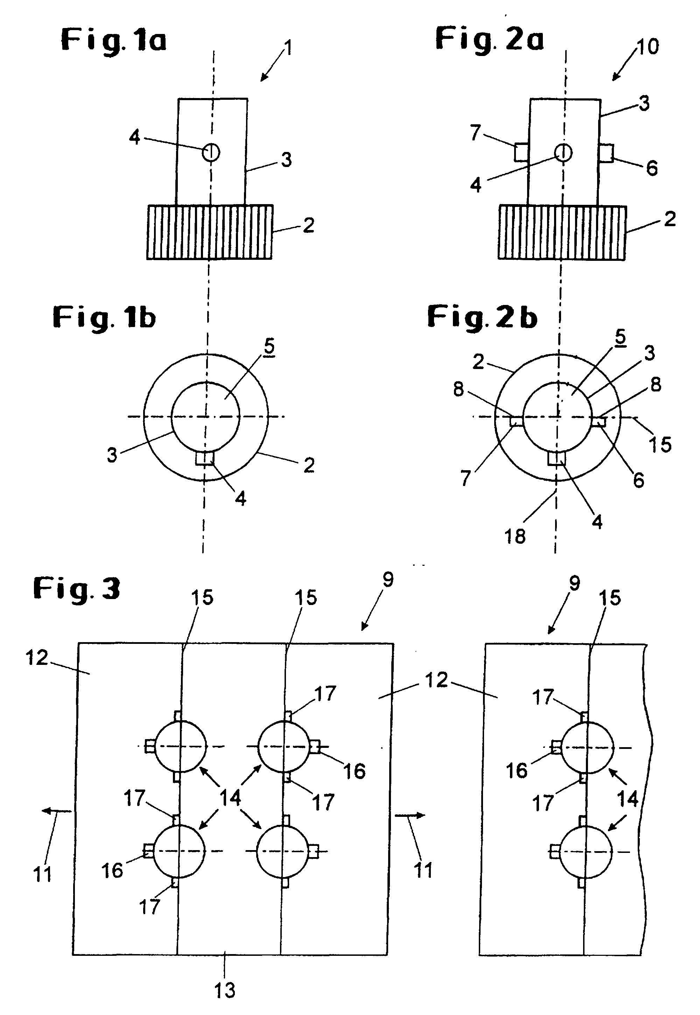

Die Fig. 1a, 1b zeigen eine zum Stand der Technik zählende Verschlußkappe 1 zum Aufstecken oder -schrauben auf das offene Ende eine Blutentnhameröhrchens einer als solche hinlänglich bekannten Blutentnahmevorrichtung (nicht dargestellt). Die Verschlußkappe 1 besteht aus einer zylindrischen Hülse 2 und einem darauf angeordneten Dom 3; dieser besitzt einen seitlich abstehenden, im Ausführungsbeispiel zylindrischen Haltenocken 4 und ist oben mit einem Gummistopfen 5 (vgl. Fig. 1b) versehen. Der Haltenocken 4 dient zur Rastverriegelung einer zur Blutentnahme auf den Dom 3 aufzusetzenden Führungshülse, die vorzugsweise eine Doppelkanüle mit an beiden Enden angeschärften Schneidkanten aufweist; beim Aufstecken der Führungshülse wird einerseits der Gummistopfen 5 des Doms 3 von der Kanüle durchstochen, andererseits greift der Haltenocken 4 in einen Axialschlitz der Führungshülse der Doppelkanüle ein, die sich danach bajonettverschlußartig auf dem Dom 3 verriegeln läßt, wobei sich aber eine eher lockere Passung, d.h. eine labile Verbindung ergibt.

Eine stabile Verbindung zwischen der Führungshülse einer Doppelkanüle und dem Dom 3 läßt sich hingegen mit der in den Figuren 2a, 2b gezeigten Verschlußkappe 10 erreichen, deren Dom 3 neben dem ersten Nocken 4, der als Vollnocken ausgebildet ist, noch mindestens einen, im Ausführungsbeispiel zwei weitere Haltenocken 6, 7 aufweist. Die Haltenocken 4 bzw. 6, 7 sind um 90° zueinander versetzt auf einer Mantelhälfte der Verschlußkappe 10, und zwar auf deren Dom 3 angeordnet; abweichend von der vollzylindrischen Form des Haltenockens 4 sind die Haltenocken 6, 7 halbzylindrisch, d.h. mit der Kontur eines Halbkreises ausgebildet, die der axial in der Mittenebene 18 (vgl. Fig. 2b) durchtrennten Symmetriehälfte des vollzylindrischen Nockens 4 entspricht, d.h. sie besitzen an ihren der nockenfreien Mantelhälfte des Doms 3 zugewandten Seiten eine ebene Abschlußfläche 8.

Die Verschlußkappen 10 werden in einer Spritzgießmaschine mit einem in Fig. 3 schematisch dargestellten Werkzeug 9 hergestellt, von denen sich in gebührendem Abstand ggf. mehrere - wie angedeutet - nebeneinander anordnen lassen. Das Werkzeug 9 besitzt einen 3-teiligen Aufbau und besteht aus äußeren, in Pfeilrichtung 11 seitlich wegbewegbaren Formbacken 12 und einer mittleren, stationären Formbacke 13. Der das geschlossene Werkzeug 9 zeigenden Fig. 3 ist zu entnehmen, daß Formnester 13 in zwei nebeneinanderliegenden Reihen angeordnet sind, die in den jeweils zwischen den beweglichen Formbacken 12 und der stationären Formbacke 13 verlaufenden Trennebenen 15 liegen. Die die Endform des herzustellenden Spritzgußteiles, d.h. der Verschlußkappe 10 festlegenden Formnester 14 sind je zur Hälfte in den beweglichen Formbacken 12 und der komplementären stationären Formbacke 13 vorgesehen.

Ausschließlich in den beweglichen Formbacken 12 sind die Kavitäten 16 bzw. 17 für den vollzylindrischen Haltenocken 4 bzw. die beiden halbzylindrischen Nocken 6, 7 eingearbeitet; die mit den Trennebenen 15 zusammenfallenden Außenflächen der stationären Formbacke 13 begrenzen hierbei die Kavitäten 17 für die beiden halbzylindrischen Haltenocken 6, 7. Die ebene Abschlußfläche 8 der Haltenocken 6, 7 ergibt sich somit aufgrund der in den Trennebenen 15 verlaufenden Begrenzung der Kavitäten 17 durch die stationäre Formbacke 13. Es ist somit das gleichzeitige Herstellen mehrerer Verschlußklappen in einem Arbeitsgang möglich, indem nämlich die 2-reihige Anordnung der Formnester 14 beinhaltet werden kann und dennoch die Entformung der fertigen Spritzgußteile bzw. Verschlußkappen 10 gewährleistet ist, weil die Kavitäten 16 bzw. 17 aller Haltenocken 4 bzw. 6, 7 ausschließlich in den beweglichen Formbacken 12 angeordnet sind.

## Patentansprüche

1. Vorrichtung zum Herstellen von Spritzgußteilen, die mit mindestens zwei Vorsprüngen (6, 7) versehen sind, umfassend mindestens ein Werkzeug (9), das in zwei zwischen jeweils einer feststehenden und einer beweglichen Formbacke (12, 13) nebeneinanderliegenden Reihen angeordnete Formnester (14) aufweist,
**dadurch gekennzeichnet,**
**daß** den Vorsprüngen (6, 7) Kavitäten (16, 17) ausschließlich in den beweglichen Formbacken (12) zugeordnet sind, wobei die Kavität (17) wenigstens des einen der Vorsprünge (6, 7) einer in den Trennebenen (15) zwischen den Formbacken (12 bzw. 13) von jeweils der feststehenden Formbacke (13) begrenzten Teilkontur einer üblichen vollständigen Kavität entspricht.

2. Spritzgußteil, erhältlich mit der Vorrichtung nach Anspruch 1,
**dadurch gekennzeichnet,**
**daß** eine das Vorderende eines Entnahmeröhrchens einer Blutentnahmevorrichtung temporär verschließende Kappe (10) zur Festlegung eines aufsteckbaren, eine Kanüle tragenden Halters zumindest zwei diametral zueinander angeordnete, als halbe Vorsprünge (6, 7) ausgebildete Haltenokken aufweist.

## Claims

1. Device for the production of injection-moulded parts, which are provided with at least two projections (6, 7), comprising at least one tool (9) which has mould nests (14) arranged in two mutually adjacent rows respectively between a stationary and a movable mould cheek (12, 13), **characterised in that** cavities (16, 17) exclusively in the movable mould cheeks (12) are associated with the projections (6, 7), wherein the cavity (17) of at least one of the projections (6, 7) corresponds with a partial contour, which is bounded in the separating planes (15) between the mould cheeks (12 or 13) by the stationary mould cheek (13) in each case, of a usual complete cavity.

2. Injection-moulded part obtainable with the device according to claim 1, **characterised in that** a cap (10), which temporarily closes the forward end of a removal tubelet of a blood removing device, for fixing a holder, which can be plugged on and carries a hollow needle, has at least two retaining dogs arranged diametrically opposite one another and constructed as half projections (6, 7).

## Revendications

1. Dispositif de production de pièces coulées par injection pourvues d'au moins deux saillies (6, 7), comportant au moins un outil (9) qui comprend des empreintes de moulage (14) agencées en deux rangées situées l'une à côté de l'autre entre une mâchoire de moulage stationnaire et une mâchoire de moulage mobile (12, 13), **caractérisé en ce que** des cavités (16, 17) exclusivement dans les mâchoires de moulage mobiles (12) sont associées aux saillies (6, 7), et la cavité (17) de l'une au moins des saillies (6, 7) correspond à un contour partiel d'une cavité complète habituelle et limité par la mâchoire de moulage stationnaire respective (13) dans les plans de séparation (15) entre les mâchoires de moulage (12 ou 13).

2. Pièce coulée par injection, susceptible d'être obtenue par le dispositif selon la revendication 1, **caractérisée en ce qu'**un capuchon (10) refermant temporairement l'extrémité avant d'un tube de prélèvement d'un dispositif de prélèvement sanguin comprend au moins deux cames de maintien agencées diamétralement l'une par rapport à l'autre et réalisées sous forme de demi-saillies (6, 7), pour la fixation d'un élément de maintien enfichable portant une canule.
